# EUROPÄISCHE PATENTSCHRIFT

(11) **EP 0 780 128 B1**
(45) Veröffentlichungstag und Bekanntmachung des Hinweises auf die Patenterteilung: **09.06.2004**
(21) Anmeldenummer: 96119699.5
(22) Anmeldetag: 09.12.1996
(51) Int. Cl.: A61K 38/02, A61K 31/44, A61K 31/275

(54) **Zubereitung, enthaltend Lipoproteine und Crotonsäureamidderivate**
Crotonic acid amide derivatives and lipoprotein containing compositions
Compositions contenant des lipoprotéines et des dérivés d'amides de l'acide crotonique

(30) Priorität: 20.12.1995 DE 19547648
(43) Veröffentlichungstag der Anmeldung: 25.06.1997
(73) Patentinhaber: Aventis Pharma Deutschland GmbH, 65929 Frankfurt am Main (DE)
(72) Erfinder: Müllner, Stefan, Dr., 65239 Hochheim (DE); Hofmann, Axel, Dr., 65929 Frankfurt (DE); Saar, Karin, 64584 Biebesheim (DE); Schorlemmer, Hans-Ulrich, Dr., 35041 Marburg (DE); Bartlett, Robert, Dr., 64291 Darmstadt (DE)

(56) Entgegenhaltungen:
- EP-A- 0 529 500
- EP-A- 0 538 783
- EP-A- 0 617 959
- EP-A- 0 665 013
- WO-A-91/17748
- LUCIEN J. ET AL: "blood distribution and single-dose pharmacokinetics of leflunomide" THERAPEUTIC DRUG MONITORING, Bd. 17, Nr. 5, Oktober 1995 (1995-10), Seiten 454-9, XP000942781

## Beschreibung

Als Lipoproteine bezeichnet man hochmolekulare, wasserlösliche Komplexe, bestehend aus Lipiden (Cholesterin, Triglyceride, Phospholipide) und einem oder mehreren ganz bestimmten Proteinen, die Lipide komplexieren können und als Apolipoproteine bezeichnet werden. Die Lipoproteine stellen die Funktionseinheit für den Transport der wasserunlöslichen Lipide im Blut dar. Der Struktur der Lipoproteine ist gemeinsam, daß sich die hydrophilen Anteile der Phospholipide und die hydrophilen Anteile der Apolipoproteine an der Oberfläche, die hydrophoben Triglyceride und Cholesterinester hingegen im Inneren der sphärischen Partikel anordnen (pseudomicellare Partikel).

Die Lipoproteine werden in verschiedenen Dichteklassen eingeteilt. Lipide besitzen eine wesentlich geringere Dichte als Proteine. Für erstere liegt das spezifische Gewicht unter 0,9 g/ml KBr, für die Proteine über 1,28 g/ml KBr. Komplexe aus Lipiden und Proteinen liegen demzufolge in ihrer Dichte zwischen den genannten Extremwerten. In der Ultrazentrifuge können die Lipoproteine in 4 Dichteklassen aufgetrennt werden.

Chylomikronen (Dichte d < 0,95 g/ml) werden im Darm gebildet und transportieren exogene Triglyceride, ihr Lipidanteil beträgt 98 - 99,5 %, der Proteinanteil 0,5 - 2 %; sie rahmen im Serum auf.

VLDL (= Very Low Density Lipoproteine = Lipoproteine sehr niedriger Dichte; d < 1,006 g/ml ) werden in der Leber gebildet und transportieren den Hauptanteil der endogenen Triglyceride und bestehen zu 85 - 90 % aus Lipiden und zu 10 - 15 % aus Protein.

IDL (Intermediate Density Lipoproteine = Lipoproteine intermediärer Dichte; d = 1,006 - 1,019 g/ml) sind bei Stoffwechselgesunden nur in sehr niedriger Konzentration nachweisbar und werden als Stoffwechselprodukte der VLDL oder Vorläufermoleküle der LDL aufgefaßt.

LDL (Low Density Lipoproteine = Lipoproteine niedriger Dichte; d = 1,019 - 1,063 g/ml) transportieren den Hauptanteil des Cholesterins im Blut. Die LDL entstehen als Stoffwechselprodukt aus den VLDL und enthalten etwa 75 % Lipide und 25 % Proteine.

HDL (= High Density Lipoproteine = Lipoproteine hoher Dichte; d = 1,063 - 1,21 g/ml) enthalten etwa 50 % Protein und 50 % Lipide und werden als Vorläufermoleküle von Darm und Leber gebildet und im Plasma ausgeformt; sie können Cholesterin aus den Zellen aufnehmen und zur Leber zurücktransportieren. Aufgrund der verschiedenen Komposition und Morphologie sowie funktioneller Eigenschaften unterscheidet man die HDL-Subfraktionen HDL₁ (1,055 - 1,085 g/ml), HDL₂ (1,063 - 1,12 g/ml) und HDL₃(1,12-1,21 g/ml).

HDL sind keine einheitliche Substanz, sondern ein heterogenes Gemisch von Makromolekülen, die sich durch Partikelgröße, chemische Zusammensetzung und physikochemische Eigenschaften unterscheiden. Referenzmethode zur Darstellung der HDL ist die Flotation in der präparativen Ultrazentrifuge (d = 1,063 - 1,21 g/ml). Der überwiegende Teil der in dieser Dichteklasse gefundenen Partikel hat folgende Eigenschaften:
a) Elektrophorese: Wanderung in α-Position,
b) Elektronenmikroskopie, sphärische Partikel mit einem Durchmesser von 80 - 120 x 10⁻¹⁰ m,
c) chemische Komposition: Apo A-I-Anteil (30-35 %), Apo A-II-Anteil (10-15 %), Apo C-Anteil (3-5 %), Phospholipid-Anteil (25-30 %), Cholesterin/Cholesterinester-Anteil (15-20 %), Triglycerid-Anteil (3 - 5 %).

Abhängig von der Isolierungstechnik und dem Gehalt von Lipoprotein (a) in verschiedenen Seren können in der HDL-Dichteklasse variable Mengen von Apo-B-enthaltenden Lipoproteinen (Lipoprotein (a), LDL) gefunden werden; andererseits sind geringe Verluste von HDL in die d > 1.21 g/ml Dichteklasse bei präparativer Ultrazentrifugation zu verzeichnen.

Die HDL-Fraktion läßt sich durch besondere Isolierungstechniken in verschiedene Subfraktionen auftrennen. Die für klinische Belange wichtigsten Unterfraktionen werden als HDL₁ (1,055 - 1,085 g/ml), HDL₂ (1,063 - 1,125 g/mt) und HDL₃ (1,125 - 1,210 g/ml) bezeichnet. Sie zeichnen sich durch unterschiedliche Lipid- und Proteinkompositionen, physikochemische und funktionelle Eigenschaften aus.

Die Unterfraktion HDL₁ läßt sich durch spezielle Trenntechniken (z.B. Zonalzentrifugation, Heparin-Affinitätschromatographie) isolieren. Sie enthält Apo E als dominierende Proteinkomponente. Unter Cholesterinfütterung nimmt bei verschiedenen Tierspezies die Konzentration dieser Fraktion stark zu; man bezeichnet diese cholesterinreduzierte HDL-Fraktion als HDL_{C}. HDL_{C} ist auch beim Menschen nach mehrwöchiger cholesterinreicher Nahrung nachweisbar.

Ungefähr 50 % der HDL-Masse sind Protein, 30 % Phospholipide, 10 bis 20 % Cholesterin- und Cholesterinester und 5 % Triglyceride. Das Lecithin/Sphingomyelin-Verhältnis beträgt 5:1, das Verhältnis verestertes Cholesterin/freies Cholesterin etwa 3:1. HDL₂ enthält etwa 60 % Lipide und 40 % Protein, während HDL₃ zu etwa 45 % aus Lipiden und zu etwa 55 % aus Proteinen besteht. Das Phosphatidylcholin/Sphingomyelin-Verhältnis von freiem zu verestertem Cholesterin ist in den HDL₂ höher als in den HDL₃. HDL_{C} enthält einen besonders hohen Anteil an Apo E und Cholesterinestern. Der Proteinanteil der HDL setzt sich aus mehreren Apolipoproteinen zusammen. Ungefähr 90 % des Proteinanteils der HDL bestehen aus Apo A-I und Apo A-II. Das Mengenverhältnis beider Apolipoproteine scheint in den Subpopulationen des HDL unterschiedlich zu sein und zum Teil von der jeweiligen Präparation mit dem Zonalrotor wurde in der HDL₂-Fraktion ein molares Apo A-I /Apo A-II Quotient von 9:1 und in der HDL₃-Fraktion ein Quotient von 2:1 gefunden.

Crotonsäureamidderivate, deren Herstellung und deren Verwendung als Arzneimittel sind bekannt aus EP 484 223, EP 529 500, EP 538 783, US 4 061 767 und EP 551 230.

Es wurde nun gefunden, daß Verbindungen der Formel I sich in der HDL-Fraktion des Blutes anreichern und Zubereitungen enthaltend High Density Lipoproteine und eine Verbindung der Formel I, zur Dosiserniedrigung der Verbindung der Formel I bei gleicher Wirksamkeit führen. Die Dosisreduzierung führt ohne verringerte Wirksamkeit zur erhöhten Sicherheit bei der therapeutischen Anwendung. Gleichzeitig können die Therapiekosten erniedrigt werden. Ferner zeigen Zubereitungen, enthaltend Lipoproteine und Verbindungen der Formel I, verlängerte Wirksamkeit bei Transplantationen.

Die Erfindung betrifft daher eine Zubereitung enthaltend mindestens
1) ein Lipoprotein,
2) eine Verbindung der Formel I 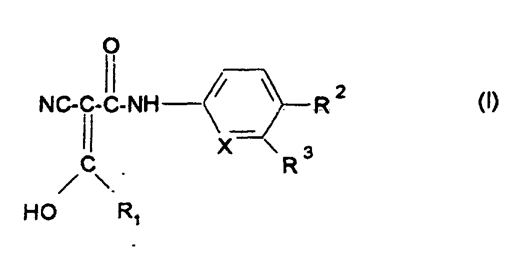 und/oder eine gegebenenfalls stereoisomere Form der Verbindung der Formel I und/oder ein physiologisch verträgliches Salz der Verbindung der Formel I und
3) einen pharmazeutischen Träger, wobei
   - R¹: für
   a) (C₁-C₄)-Alkyl
   b) (C₃-C₅)-Cycloalkyl,
   c) (C₂-C₆)-Alkenyl oder
   d) (C₂-C₆)-Alkinyl, steht,
   - R²: für
   a) -CF₃ ,
   b) -O-CF₃,
   c) -S-CF₃,
   d) -OH,
   e) -NO₂,
   f) Halogen,
   g) Benzyl,
   h) Phenyl,
   i) -O-Phenyl,
   k) -CN
   l) -O-Phenyl, ein oder mehrfach substituiert durch
      1) (C₁-C₄)-Alkyl,
      2) Halogen,
      3) -O-CF₃ oder
      4) -O-CH₃, steht,
   - R³: für
   a) (C₁-C₄)-Alkyl,
   b) Halogen, oder
   c) Wasserstoffatom steht, und
   - X: für
   a) eine -CH-Gruppe oder
   b) Stickstoffatom, steht.

Bevorzugt ist der Einsatz einer Verbindung der Formel I und/oder eine gegebenenfalls stereoisomere Form der Verbindung der Formel I und/oder ein Salz der Verbindung der Formel I, wobei
- R¹: für
a) Methyl,
b) Cyclopropyl oder
c) (C₃-C₅)-Alkinyl steht,
- R² für: CF₃ oder CN steht,
- R³ für: Wasserstoffatom oder Methyl steht, und
- X für: eine -CH-Gruppe steht.

Insbesondere bevorzugt ist die Verwendung von N-(4-Trifluormethylphenyl)-2-cyan-3-hydroxy-crotonsäureamid, 2-Cyano-3-cyclopropyl-3-hydroxy-acrylsäure-(4-cyanophenyl)-amid oder N-(4-Trifluormethylphenyl)-2-cyan-3-hydroxy-hept-2-en-6-in-carbonsäureamid.

Die Herstellung der Verbindungen der Formel I erfolgt nach bekannten Verfahren wie sie in EP 484 223; EP 538 783 oder EP 551 230 beschrieben werden.

Unter dem Begriff Alkyl, Alkenyl oder Alkinyl werden Reste verstanden deren Kohlenstoffkette geradkettig oder verzweigt sein kann. Ferner können die Alkenyl- oder Alkinyl- Reste auch mehrere Doppelbindungen beziehungsweise mehrere Dreifachbindungen enthalten. Cyclische Alkylreste sind beispielsweise 3- bis 5-gliedrige Monocyclen wie Cyclopropyl, Cyclobutyl oder Cyclopentyl. Die Ausgangsstoffe der chemischen Umsetzungen sind bekannt oder lassen sich nach literaturbekannten Methoden leicht herstellen.

Lipoproteine, die in der vorliegenden Erfindung verwendet werden, enthalten Lipide und Proteine. Bevorzugte Lipide sind natürlich vorkommende Lipide in Blut oder Serum, wie Cholesterin, Cholesterinester, Triglyceride oder Phospholipide. Bevorzugte Proteine sind lipophile Proteine, bevorzugt lipophile Proteine aus Blut oder Serum. Die Proteine können auch chemisch modifiziert sein, beispielsweise mit Polyethylenglycol modifizierte Proteine.

Das bevorzugte Protein zu Lipid-Verhältnis beträgt von etwa 20 % Protein zu 80 % Lipid, besonders bevorzugt von etwa 30 % Protein zu etwa 70 % Lipid, insbesondere bevorzugt von etwa 40 % Protein zu etwa 60 % Lipid, ganz besonders etwa 50 % Protein zu 50 % Lipid. Die Konzentration der Lipidkomponente variiert entsprechend der verwendeten Lipoproteine.

Die Lipidkomponente der Lipoproteine enthält beispielsweise von etwa 5 % bis 40 % Phospholipide, von etwa 1 % bis 20 % Triglyceride; von etwa 5 % bis 30 % Cholesterinester, oder von 1 % bis 5 % Cholesterin oder Mischungen der genannten Lipidkomponenten.

Die erfindungsgemäßen Zubereitungen enthalten von etwa 1 % bis etwa 20 % vom Trockengewicht eine Verbindung der Formel I oder ein Salz der Verbindung der Formel I.

Lipoproteine, die in der vorliegenden Erfindung verwendet werden können, sind in Tieren oder Menschen natürlich vorkommende Lipoproteine als solche, Lipoproteine, die aus nicht natürlichen Quellen stammen, oder chemisch modifizierte Lipoproteine. Bevorzugt sind Lipoproteine aus Säugetieren und insbesondere aus Menschen. Geeignete Lipoproteine können aus Säuretieren wie Rindern, Pferden der Kaninchen gewonnen werden. Besonders geeignet sind Lipoproteine aus den zu behandelnden Patienten, da autologe Lipoproteine geringe bis keine Toxizitäten und Immunogenitäten aufweisen.

Bevorzugt sind HDL Lipoproteine geeignet und können aus Säugetieren wie Rindern, Pferden oder Kaninchen gewonnen werden. Besonders geeignet ist HDL aus dem zu behandelnden Patienten, da autologes HDL geringere bis keine Toxizität und Immunogenität aufweist als HDL aus Säuretieren. Es können jedoch auch HDL-Fraktionen, Subfraktionen oder HDL-Bestandteile in der erfindungsgemäßen Zubereitung eingesetzt werden. Ferner kann das HDL enzymatisch oder chemisch modifiziert werden.

Die erfindungsgemäße Zubereitung eignet sich beispielsweise zur Behandlung von
- Immunologischen Erkrankungen
- Krebserkrankungen wie Lungenkrebs, Leukämie, Eierstockkrebs, Sarkome, Kaposi's Sarkom, Meningiom, Darmkrebs, Lymphknotenkrebs, Hirntumore, Brustkrebs, Pankreaskrebs, Prostatakrebs oder Hautkrebs
- Autoimmunerkrankungen wie systemischem Lupus erythematodes oder multipler Sklerose
- Rheumaerkrankungen
- Transplantationen oder Graft-versus-Host-Reaktionen oder Host-versus-Graft-Reaktionen
- akute, chronische oder hyperakute Abstoßungsreaktionen nach Transplantation von Organen wie Niere, Herz, Haut, Leber, Knochen, Blut oder Haare
- Erkrankungen, die durch stark proliferierende Zellen verursacht werden
- Psoriasis oder atypischer Dermatitis
- Allergie, Asthma, Urticaria, Rhinitis oder Uveitis
- Typ II-Diabetes
- zystischer Fibrose, Kolitis, Leberfibrose oder Sepsis.

Die Erfindung betrifft auch ein Verfahren zur Herstellung der Zubereitung, das dadurch gekennzeichnet ist, daß man die Verbindung der Formel I und/oder ein physiologisch verträgliches Salz der Verbindung der Formel I und HDL mit einem pharmazeutisch geeigneten und physiologisch annehmbaren Träger und gegebenenfalls weiteren geeigneten Wirk-, Zusatz- oder Hilfsstoffen in eine geeignete Darreichungsform bringt.

Die erfindungsgemäße Zubereitung kann intravenös oder parenteral appliziert werden. Geeignete flüssige galenische Darreichungsformen sind beispielsweise injizierbare Lösungen, Suspensionen oder Emulsionen.

Vorzugsweise wird die Zubereitung in Dosierungseinheiten hergestellt und verabreicht, wobei jede Einheit als aktiver Bestandteil eine bestimmte Dosis von der Verbindung der Formel I und/oder physiologisch verträgliche Salze der Verbindung der Formel I enthält. Für die Behandlung eines Patienten (70 kg), sind in frühen Phasen eine intravenöse Infusionsbehandlung von maximal 1200 mg pro Tag und in der späteren Rehabilitationsphase maximal 3 mal 300 mg pro Tag der Verbindung der Formel I und/oder der entsprechenden Salze der Verbindung der Formel I indiziert.

Die Menge der Lipoproteine, insbesondere an HDL in der Zubereitung beträgt von 0,1 ml bis 100 ml pro Tag. Die Mengenangabe ml HDL bezieht sich auf die Präparation gemäß Beispiel 1a, wobei das Serum und das HDL keine Verbindung der Formel I enthält.

Die anzuwendende Dosierung ist selbstverständlich abhängig von verschiedenen Faktoren wie dem zu behandelnden Lebewesen (d.h. Mensch oder Tier), Alter, Gewicht, allgemeiner Gesundheitszustand, dem Schweregrad der Symptome, der zu behandelnden Erkrankung, eventuellen Begleiterkrankungen (falls vorhanden), der Art der begleitenden Behandlung mit anderen Arzneimitteln oder der Häufigkeit der Behandlung. Die Dosierungen werden im allgemeinen mehrfach pro Tag und vorzugsweise einmal bis dreimal pro Tag verabreicht. Die verwen-dete Menge der Verbindung der Formel I orientiert sich hierbei an der empfohlenen Tagesdosis der jeweiligen Verbindung der Formel I und der Löslichkeit der Verbindung der Formel I in HDL. Im allgemeinen liegt die Konzentration der Verbindung der Formel I ir der Zubereitung von 10 % bis 100 % der empfohlenen Tagesdosis, bevorzugt von 20 % bis 80 %, insbesondere bei 50 %. Die geeignete Therapie mit den erfindungsgemäßen Kombinationen besteht somit z.B. in der Verabreichung von einer, zwei oder drei Einzeldosierungen der erfindungsgemäßen Zubereitung bestehend aus
1) 5 mg bis 50 mg, vorzugsweise von 10 mg bis 20 mg N-(4-Trifluormethylphenyl)-2-cyan-3-hydroxy-crotonsäureamid und
2) 0,1 ml bis 10 ml, vorzugsweise 1 ml bis 3 ml HDL,
wobei die Menge naturgemäß von der Zahl der Einzeldosierungen und auch der zu behandelnden Krankheit abhängig ist. Eine Einzeldosierung kann auch aus mehreren, gleichzeitig verabreichten Dosiereinheiten bestehen. Das Verhältnis der Verbindung der Formel I zur Menge an HDL in den Dosiereinheiten hängt von der Löslichkeit der Verbindung der Formel I in HDL ab. In der Regel werden je mg der Verbindung der Formel I von 0,01 bis 10 ml HDL eingesetzt, bevorzugt von 0,1 ml bis 5 ml HDL.

Ferner können die erfindungsgemäßen Zubereitungen auch zusammen mit anderen geeigneten Wirkstoffen, beispielsweise Antiuricopathika, Thrombocytenaggregationshemmern, Analgetika, steroidalen oder nichtstereoidalen Antiphlogistika oder immunsuppressiven Verbindungen wie Cylosporin A, FK 506 oder Rapamycin eingesetzt werden.

### Beispiel 1

### N-(4-Trifluormethylphenyl)-2-cyan-3-hydroxy-crotonsäureamid (Verbindung 1) in Lipoproteinfraktionen aus Humanserum

Drei männliche freiwillige Versuchspersonen erhalten an drei aufeinander folgenden Tagen oral je 100 mg N-(4-Trifluormethylphenyl)-2-cyan-3-hydroxycrotonsäureamid in Pulverform. Etwa vier Stunden nach der letzten Medikation werden je 100 ml Blut entnommen. Aus diesem Blut wird durch Zentrifugation Serum gewonnen.

### a) Präparation der Lipoprotein-Fraktionen

Das Serum jeder Versuchsperson wird durch Einrühren von festem KBr, wobei je ml Serum 0,0199 g KBr eingesetzt werden, auf eine Dichte von 1,020 g/ml gebracht. Nach 20stündiger Zentrifugation bei 4°C und 140 000 xg setzt sich eine "very low density lipoprotein" (VLDL) enthaltende milchig-weiße Lipoproteinschicht auf dem Serum ab.
Nach vorsichtigem Abpipettieren dieser Schicht wird das verbliebene Serum mit 0,0628 g KBr/ml Serum vermischt, wobei eine Dichte von 1,063 g/ml erreicht wird. Nach 20stündiger Zentrifugation bei 4°C und 140 000 xg wird die bräunliche "low density lipoprotein" LDL-Schicht vom Serum abgenommen. Das verbliebene Serum wird mit 0,234 g KBr/ml Serum vermischt, wobei eine Dichte von 1,21 g/ml erreicht wird. Nach 20stündiger Zentrifugation bei 4°C und 140 000 xg wird die "high density lipoprotein" HDL-Schicht abgenommen. Die erhaltenen HDL-Fraktionen werden in einen Dialyseschlauch mit einer Ausschlußgrenze von 6000 - 8000 kD gegeben und gegen einen Puffer pH 7,4 für 72 Stunden dialysiert. Alle 24 Stunden wird der Puffer 2 bis 3 mal gewechselt. Die dialysierten HDL-Fraktionen können sofort verwendet werden oder werden bei -80°C gelagert. Nach der Dialyse wird eine Proteinbestimmung der dialysierten HDL-Fraktionen durchgeführt. Es ergibt sich ein Wert von etwa 20 mg Protein pro ml dialysierte HDL-Fraktion.

### b) Extraktion der Verbindung 1 aus den Proben

Dazu werden aus allen Lipoproteinfraktionen und zusätzlich vom Ausgangsplasma je 500 µl abgenommen und langsam zu je 1200 µl Acetonitril zugetropft. Die Proben werden 10 min im Ultraschallbad behandelt und 10 min bei 14 000 xg abzentrifugiert.
Bei den HDL-Proben bilden sich zwei Phasen, von denen die obere weiter verarbeitet wird. Von allen Proben wird der Überstand abgenommen und über Nacht im Trockenschrank bei 60°C eingedampft. Am nächsten Tag werden die Proben in je 100 µl Methanol aufgenommen und analysiert.

### c) Analysenmethode

Die Proben werden mit Hochdruckflüssigchromatographie (HPLC) analysiert.
- Laufmittel:: 0,1 M H₃PO₄: Acetonitril 1:1
- Säule:: LiChrospher 100 RP 18, 250 x 4 mm, 5 µ, Fa. Merck AG
- Flußrate:: 1,5 ml/min
- Injektion:: 50 µl je Probe
- Detektion:: mit Diode-Array-Detektor

### d) Ergebnis

Es wird je eine Standardkurve mit der Verbindung 1 aufgenommen. Durch Vergleich der UV-Spektren mit der in den Proben gefundenen Substanz ergibt sich, daß im Plasma ausschließlich Verbindung 1 vorhanden ist. Die Gesamtmenge der im Plasma nachgewiesenen Substanz ist bei den drei Probanden wie folgt: Versuchsperson 1 (VP1) bei 32,00 µg/ml, bei VP2 bei 9,61 µg/ml und bei VP3 bei 36,27 µg/ml.
Außer im Voliplasma kann nur in den HDL-Fraktionen Verbindung 1 gefunden werden. Hier wird bei VP1 1,55 µg/ml, bei VP2 2,39 µg/ml und bei VP3 1,03 µg/ml der Verbindung 1 festgestellt.

### Beispiel 2

### Einfluß von HDL auf die Wirkung von Verbindung 1 im Testmodell akute experimentelle allergische Encephalomyelitis (EAE)

Die Versuchsdurchführung erfolgt wie in H.W. Schorlemmer und R.R. Bartlett (Agents Actions, 41, Special Conference Issue: C271-C273, (1994)) beschrieben. 10 mg der Verbindung 1 werden in 1 ml der dialysierten HDL-Fraktion, gelöst. Das HDL wird wie in Beispiel 1 beschrieben aus Rattenserum präpariert. Dazu werden 100 mg der Verbindung 1 in 10 ml dialysierte HDL-Fraktion gegeben und über Nacht auf dem Magnetrührer bei mittlerer Rührgeschwindigkeit und 4 °C gerührt. Dann wird 3 mal für 30 sec mit Ultraschall suspendiert und weitere 4 Stunden gerührt. Zum Schluß wird die Zubereitung 15 min im Ultraschallbad behandelt. Die Zubereitung wird den Ratten intravenös verabreicht.

Verbindung 1 ohne HDL wird in einer Konzentration von 10 mg/ml Lösungsmittel (1 % Carboxymethylcellulose in Wasser) den Ratten intravenös verabreicht. Die Konzentrationsangaben beziehen sich in Tabelle jeweils auf 1 kg Lebengewicht einer Ratte. Die Kontrollgruppe erhält nur Lösungsmittel (1 % Carboxymethylcellulose in Wasser) intravenös verabreicht. Es werden jeweils 5 Lewis Ratten pro Experiment eingesetzt. Tabelle 1 zeigt die Ergebnisse.

**Tabelle 1**

| Tage nach Injektion | Verbindung 1 | | | Kontrolle | HDL 0,2 ml/kg Ratte | 0,2 ml HDL + 2 mg Verbindung 1 /kg Ratte |
|---|---|---|---|---|---|---|
| | 20 mg/kg Ratte | 10 mg/kg Ratte | 2 mg/kg Ratte | | | |
| 8 | 0 | 0 | 0 | 0 | 0 | 0 |
| 9 | 0 | 0 | 0 | 0 | 0 | 0 |
| 10 | 0 | 0 | 0 | 0,1 ± 0,3 | 0,2 ± 0,5 | 0 |
| 11 | 0 | 0 | 0,5 ± 1,1 | 1,3 ± 0,8 | 1,3 ± 0,9 | 0 |
| 12 | 0 | 0 | 1,4 ± 1,5 | 3,2 ± 0,7 | 2,3 ± 1,1 | 0 |
| 13 | 0 | 0 | 2,7 ± 1,4 | 4,6 ± 0,5 | 3,6 ± 0,8 | 0 |
| 14 | 0 | 0 | 3,8 ± 1,6 | 5,8 ± 0,9 | 4,8 ± 0,4 | 0,2 ± 0,5 |
| 15 | 0 | 0,7 ± 0,8 | 4,5 ± 1,5 | 6,6 ± 0,8 | 5,7 ± 1,0 | 0,4 ± 1,0 |
| 16 | 0,3 ± 0,5 | 2,4 ± 1,0 | 4,5 ± 1,4 | 6,8 ± 0,7 | 6,8 ± 0,5 | 1,5 ± 1,3 |
| 17 | 0,6 ± 0,8 | 3,6 ± 0,7 | 3,2 ± 2,3 | 7,0 ± 0 | 7,0 ± 0 | 2,0 ± 1,4 |
| 18 | 1,2 ± 0,9 | 3,8 ± 0,6 | 2,9 ± 2,4 | | | 2,6 ± 1,9 |
| 19 | 1,8 ± 0,9 | 3,3 ± 0,5 | 2,6 ± 2,5 | | | 2,5 ± 1,6 |
| 20 | 2,2 ± 1,2 | 1,9 ± 0,4 | 2,5 ± 2,5 | | | 1,8 ± 1,4 |
| 21 | 2,3 ± 1,4 | 1,6 ± 0,5 | 2,4 ± 2,6 | | | 1,4 ± 1,2 |
| 22 | 2,3 ± 1,5 | 1,2 ± 0,4 | 2,4 ± 2,7 | | | 1,4 ± 1,2 |
| 23 | 2,0 ± 1,9 | 1,0 ± 0,4 | 2,2 ± 2,8 | | | 0,9 ± 0,7 |
| 24 | 1,4 ± 2,1 | 0,8 ± 0,7 | 2,0 ± 2,8 | | | 0,5 ± 0,7 |
| 25 | 1,2 ± 2,1 | 0,2 ± 0,4 | 1,7 ± 2,8 | | | 0,2 ± 0,4 |
| 26 | 0,9 ± 2,3 | 0 | 1,4 ± 3,0 | | | 0 |
| 27 | 0,7 ± 2,2 | | 1,4 ± 3,0 | | | |

Die Ergebnisse zeigen, daß alle Tiere in der Kontrollgruppe und 0,2 ml HDL-Gruppe innerhalb von 17 Tagen sterben. In den Behandlungsgruppen 10 mg Verbindung 1 und 20 mg Verbindung 1 erfolgt ein verzögertes Einsetzen der Erkrankung und das Verschwinden der Symptome nach etwa 26 Tagen. In der Behandlungsgruppe 2 mg Verbindung 1 treten deutlich stärkere Symptome auf und das Abklingen der Symptome erfolgt langsamer. In der Behandlungsgruppe 0,2 ml HDL und 2 mg Verbindung 1 - erfindungsgemäße Zubereitung - erfolgt ein verzögertes Einsetzen der Erkrankung und das Verschwinden der Symptome nach 25 Tagen. Die Verzögerung der Erkrankung in der Behandlungsgruppe 0,2 ml HDL und 2 mg Verbindung 1 entspricht etwa der Behandlungsgruppe 10 mg Verbindung 1 und die Schwere der aufgetretenen Symptome entspricht etwa der Behandlungsgruppe 20 mg Verbindung 1. Daher führt die erfindungsgemäße Zubereitung zu einer Dosiserniedrigung der Verbindung 1 bei gleich guter Wirksamkeit.

### Beispiel 3

### N-(4-Trifluormethylphenyl)-2-cyan-3-hydroxy-hept-2-en-6-in-carbonsäureamid Natriumsalz (Verbindung 2)

50 g (0,15 mol) N-(4-Trifluormethylphenyl)-2-cyano-3-hydroxy-hept-2-en-6-in-carbonsäureamid werden in einem Zweiphasensystem aus 50 ml 5 N Natronlauge und 500 ml Ethylacetat gelöst, die organische Phase abgetrennt, zweimal mit wenig Wasser gewaschen, über Natriumsulfat getrocknet und eingeengt. Der ölige Rückstand wird mit 500 ml tertiär-Butylmethylether aufgenommen und zur vollständigen Kristallisation 4 Stunden (h) bei Raumtemperatur gerührt, filtriert und unter vermindertem Druck getrocknet. Zur vollständigen Entfernung von Lösemittelresten wird das kristalline Produkt in 500 ml Toluol 10 min unter Rückfluß suspendiert, unter Rühren abgekühlt, erneut abgesaugt und unter vermindertem Druck getrocknet. Ausbeute: 41,1 g (77 %) vom Schmelzpunkt ≥ 244°C Zersetzung (Zers.).

| C ₁₅ H ₁₀ F ₃ N ₂ O ₂ Na (330,24 g/mol): | | | | | |
|---|---|---|---|---|---|
| berechnet | C: 54,0 | H: 3,5 | N: 8,4 | Na: 6,88 | (ber. für 1,1 % Wasser) |
| gefunden | C: 54,4 | H: 3,4 | N: 8,4 | Na: 6,5 | Wasser: 1,1 % |

### Beispiel 4

### 2-Cyano-3-cyclopropyl-3-hydroxy-acrylsäure-(4-cyanophenyl)-amid Natriumsalz (Verbindung 3)

15 g (0,059 mol) 2-Cyano-3-cyclopropyl-3-hydroxy-acrylsäure-(4-cyanophenyl)-amid werden in 120 ml Wasser und 100 ml Aceton suspendiert und durch Zugabe von 60 ml 1 N NaOH in Lösung gebracht. Nach Filtration von Spuren an ungelöstem Material wird am Rotationsverdampfer unter vermindertem Druck auf etwa 200 ml aufkonzentriert, und bei 0°C über Nacht kristallisiert, abgesaugt und unter vermindertem Druck getrocknet.
Ausbeute: 13 g, Schmp. > 280°C.

| C₁₄H₁₀N₃Na (275,24): | | | | |
|---|---|---|---|---|
| berechnet | C: 60,7 | H: 3,7 | N: 15,2 | (ber. für 0.7 % Wasser) |
| gefunden | C: 60,8 | H: 3,6 | N: 15,3 | Wasser: 0,7 % |

### Beispiel 5

### Einfluß von HDL im Testmodell akute Transplantationsabstoßung

Die Versuchsdurchführung erfolgt wie in H.U. Schorlemmer et al. (Transplant. Proceeding, Band 25, Nr. 1 (1993), Seiten 763 - 767) beschrieben. Als Transplantspender dienen weibliche Dark-Agouti-Ratten (RTI*avl); Empfängerratten sind Lewis-Ratten (RTI*I) mit einem Körpergewicht von etwa 170 g. Die Ratten werden von Charles River-Wega, Sulzfeld, Deutschland, zur Verfügung gestellt.

Hautstücke vom Schwanz, Größe etwa 0,5 x 1,0 cm, werden von den Dark Agouti-Ratten (DA) auf den Schwanz der Lewis-Ratten (LEW) übertragen. Je Behandlungsgruppe werden 8 Tiere eingesetzt. Abstoßung ist definiert als transplantierte Haut mit harter Konsistenz und rotbrauner Farbe. Die Verbindungen 1, 2 und 3 sowie die HDL-Fraktionen werden wie in Beispiel 2 vorbereitet und den Tieren intravenös (i.v.) verabreicht. Die Behandlung erfolgt mit den Präparaten am Tag der Transplantation und den neun (9) folgenden Tagen je einmal täglich.

Tabelle 2 zeigt die Ergebnisse.

**Tabelle 2**

| Applizierte Verbindungen | Tag an dem Abstoßung des Transplantats erfolgt | durchschnittliche Abstoßungszeit (Tage; x ± SD) |
|---|---|---|
| Kontrolle | 7,7,7,8,8 | 8,3 ± 1,2 |
| 0,4 ml HDL | 9, 9, 9 | |
| Verbindung 1 | 18, 18, 18, 19, 20 | 19,4 ± 1,3 |
| 2,5 mg/kg Ratte/Tag | 20, 21, 21 | |
| 0,4 ml HDL + 2,5 mg Verbindung 1 / kg Ratte/Tag | 32, 32, 33, 33, 33 | 32,9 ± 0,6 |
| | 33, 33, 34 | |
| Verbindung 1 | 23, 23, 23, 23, 23 | 23,8 ± 1,4 |
| 5 mg / kg Ratte / Tag | 24, 26, 26 | |
| 0,4 ml HDL + 5 mg Verbindung 1 / kg Ratte / Tag | 34, 34, 35, 35, 36, 36, 37, 37 | 35,5 ± 1,2 |
| Verbindung 2 | 12, 13, 13, 13, 14 | 14,5 ± 2,3 |
| 2,5 mg /kg Ratte / Tag | 15, 18, 18 | |
| 0,4 ml HDL + 2,5 mg Verbindung 2 / kg Ratte / Tag | 21, 23, 24, 24, 25 | 24,1 ± 1,6 |
| | 25, 25, 26 | |
| Verbindung 2 | 15, 16, 18, 18, 18 | 18,0 ± 1,9 |
| 5 mg / kg Ratte / Tag | 19, 19, 21 | |
| 0,4 ml HDL + 5 mg Verbindung 2 / kg Ratte / Tag | 25, 25, 27, 27, 29 | 28,0 ± 2,3 |
| | 30, 30, 31 | |
| Verbindung 3 | 13, 13, 14, 14, 16 | 15,4 ± 2,1 |
| 2,5 mg / kg Ratte / Tag | 17, 18, 18 | |
| 0,4 mg HDL + 2,5 mg Verbindung 3 / kg Ratte / Tag | 21, 22, 22, 22, 23 | 22,6 ± 1,1 |
| | 23, 24, 24 | |
| Verbindung 3 | 16, 16, 17, 18, 19 | 18,8 ± 2,4 |
| 5 mg / kg Ratte / Tag | 20, 22, 22 | |
| 0,4 ml HDL + 5 mg Verbindung 3 / kg Ratte / Tag | 24, 24, 25, 25, 26 | 26,1 ± 2,0 |
| | 27, 29, 29 | |

Die Tabelle 2 zeigt, daß Zubereitungen, enthaltend HDL und eine Verbindung der Formel I, den Zeitpunkt der Abstoßung deutlich verlängern.

### Beispiel 6

Einfluß von LDL im Testmodell akute Transplantationsabstoßung LDL wird wie in Beispiel 1a beschrieben aus Rattenserum gewonnen. Die Versuchsdurchführung erfolgt wie in Beispiel 5 beschrieben. Die Behandlung erfolgt mit den Präparaten am Tag der Transplantation und an den folgenden 14 Tagen je einmal täglich.
Tabelle 3 zeigt die Ergebnisse.

**Tabelle 3**

| Applizierte Verbindungen | Tag an dem Abstoßung des Transplantats erfolgt | durchschnittliche Abstoßungszeit (Tage; x ± SD) |
|---|---|---|
| Kontrolle | 13, 14, 14, 15, 15, 15, 15, 16, 16, 16 | 14,9 ± 1,0 |
| 0,4 ml LDL | | |
| Verbindung 1 | 22, 23, 23, 23, 23, 24, 24, 25, 25, 26 | 23,8 ± 1,2 |
| 2,5 mg/kg Ratte/Tag | | |
| 0,4 ml LDL + 2,5 mg Verbindung 1 / kg Ratte/Tag | 35, 36, 36, 37, 37, 37, 37, 38, 38, 38 | 36,9 ± 1,0 |
| Verbindung 1 | 26, 27, 27, 28, 28, 29, 29, 29, 30, 30 | 28,3 ± 1,3 |
| 5 mg / kg Ratte / Tag | | |
| 0,4 ml LDL + 5 mg Verbindung 1 / kg Ratte / Tag | 40, 40, 40, 40, 41, 41, 41, 41, 42, 42 | 40,8 ± 0,8 |
| Verbindung 2 | 18, 18, 19, 19, 19, 20, 20, 21, 21, 21 | 19,6 ± 1,2 |
| 2,5 mg /kg Ratte / Tag | | |
| 0,4 ml LDL + 2,5 mg Verbindung 2 / kg Ratte / Tag | 28, 28, 28, 29, 30, 31, 31, 31, 31, 32 | 29,9 ± 1,5 |
| Verbindung 2 | 22, 23, 24, 24, 24, 24, 24, 25, 25, 25 | 24,1 ± 1,0 |
| 5 mg / kg Ratte / Tag | | |
| 0,4 ml LDL + 5 mg Verbindung 2 / kg Ratte / Tag | 35, 35, 36, 36, 36, 37, 37, 37, 37, 38 | 36,4 ± 1,0 |
| Verbindung 3 | 19, 19, 19, 20, 20, 21, 21, 21, 21, 22 | 20,3 ± 1,1 |
| 2,5 mg / kg Ratte / Tag | | |
| 0,4 mg LDL + 2,5 mg Verbindung 3 / kg Ratte / Tag | 28, 28, 29, 29, 29, 30, 30, 31, 31, 32 | 29,7 ± 1,3 |
| Verbindung 3 | 23, 23, 23, 24, 24, 24, 24, 24, 25, 25 | 23,9 ± 0,7 |
| 5 mg / kg Ratte / Tag | | |
| 0,4 ml LDL + 5 mg Verbindung 3 / kg Ratte / Tag | 34, 34, 34, 35, 35, 35, 36, 36, 36, 37 | 35,2 ± 1,0 |

Die Tabelle 3 zeigt, daß Zubereitungen, enthaltend LDL und eine Verbindung der Formel I, den Zeitpunkt der Abstoßung deutlich verlängern.

## Patentansprüche

1. Zubereitung, enthaltend mindestens
1) ein Lipoprotein,
2) eine Verbindung der Formel I 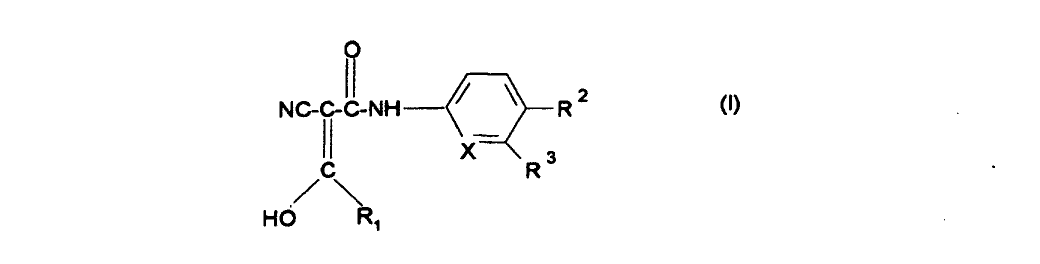 und/oder eine gegebenenfalls stereoisomere Form der Verbindung der Formel I und/oder ein physiologisch verträgliches Salz der Verbindung der Formel I, und
3) einen pharmazeutisch verträglichen Träger, wobei
R¹ für
a) (C₁-C₄)-Alkyl,
b) (C₃-C₅)-Cycloalkyl,
c) (C₂-C₆)-Alkenyl oder
d) (C₂-C₆)-Alkinyl, steht,
R² für
a) -CF₃ ,
b) -O-CF₃,
c) -S-CF₃,
d) -OH,
e) -NO₂,
f) Halogen,
g) Benzyl,
h) Phenyl,
i) -O-Phenyl,
k) -CN oder
l) -O-Phenyl, ein oder mehrfach substituiert durch
1) (C₁-C₄)-Alkyl,
2) Halogen,
3) -O-CF₃ oder
4) -O-CH₃, steht,
R³ für
a) (C₁-C₄)-Alkyl,
b) Halogen oder
c) Wasserstoffatom steht, und
X für
a) eine -CH-Gruppe oder
b) Stickstoffatom, steht.

2. Zubereitung gemäß Anspruch 1, wobei man eine Verbindung der Formel I und/oder eine gegebenenfalls stereoisomere Form der Verbindung der Formel I und/oder ein Salz der Verbindung der Formel I einsetzt, wobei
R¹ für
a) Methyl,
b) Cyclopropyl oder
c) (C₃-C₅)-Alkinyl steht,
R² für CF₃ oder CN steht,
R³ für Wasserstoffatom oder Methyl steht, und
X für eine -CH-Gruppe steht.

3. Zubereitung gemäß der Ansprüche 1 oder 2, wobei man N-(4-Trifluormethylphenyl)-2-cyan-3-hydroxy-crotonsäureamid, 2-Cyano-3-cyclopropyl-3-hydroxy-acrylsäure-(4-cyanophenyl)-amid oder N-(4-Trifluormethylphenyl)-2-cyan-3-hydroxy-hept-2-en-6-in-carbonsäureamid einsetzt.

4. Zubereitung gemäß einem oder mehreren der Ansprüche 1 bis 3, **dadurch gekennzeichnet, daß** als Lipoprotein Low Density Lipoproteine, eine High Density Lipoprotein-Fraktion, High Density Lipoprotein - Subfraktion oder ein High Density Lipoprotein-Bestandteil eingesetzt wird.

5. Zubereitung gemäß Anspruch 4, **dadurch gekennzeichnet, daß** als Lipoprotein HDL₁, HDL₂, HDL₃, HDL_{c}, Apo E, Apo A-I, Apo A-II oder Mischungen derselben eingesetzt werden.

6. Zubereitung gemäß einem oder mehreren der Ansprüche 1 bis 5, **dadurch gekennzeichnet, daß** zusätzliche Wirkstoffe aus der Gruppe Antiuricopathika, Thrombozytenaggregationshemmer, Analgetika, steroidale Antiphlogistika, nicht steroidale Antiphlogistika und immunsuppressive Verbindungen enthalten sind.

7. Zubereitung gemäß Anspruch 6, **dadurch gekennzeichnet, daß** Cylosporin A, FK 506 oder Rapamycin enthalten sind.

8. Verwendung der Zubereitung gemäß der Ansprüche 1 bis 7 zur Herstellung eines Arzneimittels zur Behandlung von immunologischen Erkrankungen.

9. Verwendung gemäß Anspruch 8 zur Behandlung von akuten immunologischen Erkrankungen wie Sepsis oder Allergie, Graft-versus-Host-Reaktionen, Host-versus-Graft-Reaktionen, von akuten, chronischen oder hyperakuten Abstoßungsreaktionen nach Transplantation von Organen wie Niere, Herz, Haut, Leber, Knochen, Blut oder Haaren, von Autoimmunerkrankungen wie rheumatoider Arthritis, systemischem Lupus erythematodes oder multipler Sklerose, Psoriasis, atypischer Dermatitis, Asthma, Urtikaria, Rhinitis, Uveitis, Typ II-Diabetes, zystischer Fibrose, Kolitis, Leberfibrose, Krebserkrankungen wie Lungenkrebs, Leukämie, Eierstockkrebs, Sarkome, Kaposi's Sarkom, Meningiom, Darmkrebs, Lymphknotenkrebs, Hirntumore, Brustkrebs, Pankreaskrebs, Prostatakrebs oder Hautkrebs.

10. Verfahren zur Herstellung der Zubereitung gemäß der Ansprüche 1 bis 7, **dadurch gekennzeichnet, daß** man High Density Lipoproteine und eine Verbindung der Formel I und einen pharmazeutischen Träger zu einer pharmazeutischen Darreichungsform verarbeitet.

## Claims

1. A preparation containing at least
1) a lipoprotein,
2) a compound of the formula I 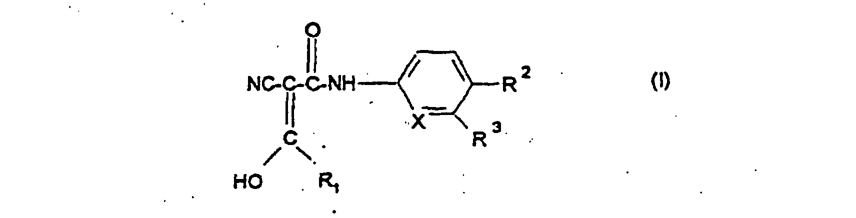 and/or an optionally stereoisomeric form of the compound of the formula I and/or a physiologically tolerable salt of the compound of the formula I and
3) a pharmaceutically tolerable excipient, where
R¹ is
a) (C₁-C₄)-alkyl
b) (C₃-C₅)-cycloalkyl,
c) (C₂-C₆)-alkenyl or
d) (C₂-C₆)-alkynyl,
R² is
a) -CF₃ ,
b) -O-CF₃,
c) -S-CF₃,
d) -OH,
e) -NO₂,
f) halogen,
g) benzyl,
h) phenyl,
i) -O-phenyl,
k) -CN or
l) -O-phenyl, mono- or polysubstituted by
1) (C₁-C₄)-alkyl,
2) halogen,
3) -O-CF₃ or
4) -O-CH₃,
R³ is
a) (C₁-C₄)-alkyl,
b) halogen, or
c) a hydrogen atom, and
X is
a) a -CH group or
b) a nitrogen atom.

2. A preparation as claimed in claim 1, in which a compound of the formula I and/or an optionally stereoisomeric form of the compound of the formula I and/or a salt of the compound of the formula I is employed, where
R¹ is
a) methyl,
b) cyclopropyl or
c) (C₃-C₅)-alkynyl,
R² is CF₃ or CN,
R³ is a hydrogen atom or methyl, and
X is a -CH group.

3. A preparation as claimed in claim 1 or 2, N-(4-trifluoromethylphenyl)-2-cyano-3-hydroxycrotonamide, 2-cyano-3-cyclopropyl-3-hydroxy-N-(4-cyanophenyl)acrylamide or N-(4-trifluoromethylphenyl)-2-cyano-3-hydroxyhept-2-en-6-ynecarboxamide being employed.

4. A preparation as claimed in one or more of claims 1 to 3, wherein the lipoprotein employed is low density lipoproteins, a high density lipoprotein fraction, high density lipoprotein subfraction or a high density lipoprotein constituent.

5. A preparation as claimed in claim 4, wherein the lipoprotein employed is HDL₁, HDL₂, HDL₃, HDL_{c}, Apo E, Apo A-I, Apo A-II or a mixture thereof.

6. A preparation as claimed in one or more of claims 1 to 5, which contains additional active compounds from the group consisting of antiuricopathics, platelet aggregation inhibitors, analgesics, steroidal antiinflammatories, nonsteroidal antiinflammatories and immunosuppressive compounds.

7. A preparation as claimed in claim 6, which contains cyclosporin A, FK 506 or rapamycin.

8. The use of the preparation as claimed in claims 1 to 7 for the manufacture of a pharmaceutical for the treatment of immunological disorders.

9. The use as claimed in claim 8 for the treatment of acute immunological disorders such as sepsis or allergy, graft-versus-host reactions, host-versus-graft reactions, of acute, chronic or hyperacute rejection reactions after transplantation of organs such as kidney, heart, skin, liver, bone, blood or hair, of autoimmune disorders such as rheumatoid arthritis, systemic lupus erythematosus or multiple sclerosis, psoriasis, atypic dermatitis, asthma, urticaria, rhinitis, uveitis, type II diabetes, cystic fibrosis, colitis, fibrosis of the liver, carcinomatous disorders such as lung cancer, leukemia, ovarian cancer, sarcoma, Kaposi's sarcoma, meningioma, colonic cancer, lymph node cancer, brain tumors, breast cancer, pancreatic cancer, prostatic cancer or skin cancer.

10. A process for the manufacture of the preparation as claimed in claims 1 to 7, which comprises processing high density lipoproteins and a compound of the formula I and a pharmaceutical excipient to give a pharmaceutical administration form.

## Revendications

1. Préparation contenant au moins
1) une lipoprotéine,
2) un composé de formule I 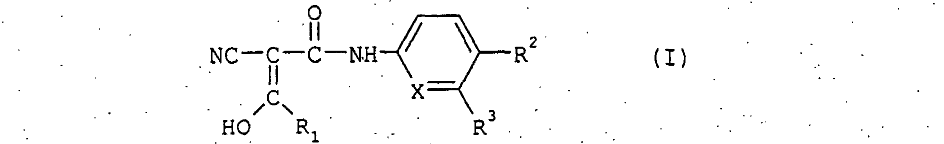 et/ou une forme éventuellement stéréoisomère du composé de formule I et/ou un sel compatible d'un point de vue physiologique du composé de formule I, et
3) un excipient acceptable d'un point de vue pharmaceutique, où
R¹ désigne un groupe
a) alkyle en C₁-C₄,
b) cycloalkyle en C₃-C₅,
c) alcényle en C₂-C₆, ou
d) alcynyle en C₂-C₆,
R² est un groupe
a) -CF₃,
b) -O-CF₃,
c) -S-CF₃,
d) -OH,
e) -NO₂,
f) halogéno,
g) benzyle,
h) phényle,
i) -O-phényle,
k) -CN ou
l) -O-phényle, substitué une ou plusieurs fois par un ou des substituants
1) alkyle en C₁-C₄,
2) halogéno,
3) -O-CF₃, ou
4) -O-CH₃,
R³ désigne un groupe
a) alkyle en C₁-C₄,
b) halogéno, ou
c) un atome d'hydrogène, et
X est
a) un groupe -CH- ou
b) un atome d'azote.

2. Préparation selon la revendication 1, dans laquelle on utilise un composé de formule I et/ou une forme éventuellement stéréoisomère du composé de formule I et/ou un sel du composé de formule I, dans laquelle
R¹ désigne un groupe
a) méthyle,
b) cyclopropyle ou
c) alcynyle en C₃-C₅, et
R² est CF₃ ou CN,
R³ est un atome d'hydrogène ou le groupe méthyle, et X est un groupe -CH-.

3. Préparation selon les revendications 1 ou 2, dans laquelle on utilise le N-(4-trifluorométhylphényl)-2-cyano-3-hydroxy-crotonamide, le (4-cyanophényl)-amide de l'acide 2-cyano-3-cyclopropyl-3-hydroxy-acrylique ou le N-(4-trifluorométhylphényl)-2-cyano-3-hydroxy-hept-2-ène-6-yne-carboxamide.

4. Préparation selon l'une ou plusieurs des revendications 1 à 3, **caractérisée en ce qu'**on utilise en tant que lipoprotéine des lipoprotéines basse densité, une fraction de lipoprotéine haute densité, une sous-fraction de lipoprotéine haute densité ou un constituant de lipoprotéine haute densité.

5. Préparation selon la revendication 4, **caractérisée en ce qu'**on utilise en tant que lipoprotéine le HDL₁, le HDL₂, le HDL₃, le HDL_{c}, l'Apo E, l'Apo A-I, l'Apo A-II ou des mélanges de ceux-ci.

6. Préparation selon l'une ou plusieurs des revendications 1 à 5, **caractérisée en ce qu'**elle contient des principes actifs additionnels choisis dans l'ensemble comprenant les antigoutteux, les inhibiteurs de l'agrégation thrombocytaire, les analgésiques, les antiphlogistiques stéroïdiens, les antiphlogistiques non stéroïdiens et les composés immunosuppresseurs.

7. Préparation selon la revendication 6, **caractérisée en ce qu'**elle contient de la cyclosporine A, du FK 506 ou de la rapamycine.

8. Utilisation de la préparation selon les revendications 1 à 7 pour préparer un médicament destiné au traitement de maladies immunologiques.

9. Utilisation selon la revendication 8 pour le traitement de maladies immunologiques aiguës telles que la sepsie ou l'allergie, les réactions greffon contre l'hôte, les réactions hôte contre le greffon, des réactions aiguës, chroniques ou hyper-aiguës de rejet après transplantation d'organes tels que les reins, le coeur, la peau, le foie, les os, le sang ou les cheveux, de maladies autoimmunes telles que la polyarthrite rhumatoïde, le lupus érythémateux disséminé ou la sclérose en plaques, le psoriasis, la dermatite atypique, l'asthme, l'urticaire, la rhinite, l'uvéite, le diabète de type II, la mucoviscidose, la colite, la fibrose hépatique, les maladies cancéreuses telles que le cancer des poumons, la leucémie, le cancer des ovaires, les sarcomes, le sarcome de Kaposi, le méningiome, le cancer de l'intestin, le cancer des ganglions lymphatiques, les tumeurs cérébrales, le cancer du sein, le cancer du pancréas, le cancer de la prostate ou le cancer de la peau.

10. Procédé de fabrication de la préparation selon les revendications 1 à 7, **caractérisé en ce qu'**on met en oeuvre pour obtenir une forme galénique pharmaceutique des lipoprotéines haute densité et un composé de formule I et un excipient pharmaceutique.
